# EUROPEAN PATENT SPECIFICATION

(11) **EP 3 449 890 B1**
(45) Date of publication and mention of the grant of the patent: **29.07.2020**
(21) Application number: 18190182.8
(22) Date of filing: 22.08.2018
(51) Int. Cl.: A61H 23/02, A61N 1/36, A61N 1/32, A61B 18/12, A61F 7/00, A61N 1/06

(54) **BEAUTY APPARATUS**
SCHÖNHEITSVORRICHTUNG
APPAREIL DE SOINS DE BEAUTÉ

(30) Priority: 29.08.2017 JP 2017163960
(43) Date of publication of application: 06.03.2019
(73) Proprietor: Panasonic Intellectual Property Management Co., Ltd., Osaka-shi, Osaka 540-6207 (JP)
(72) Inventor: IBUKI, Yasuo, Osaka-shi, Osaka 540-6207 (JP); TAKECHI, Mitsuru, Osaka-shi, Osaka 540-6207 (JP)
(74) Representative: Grünecker Patent- und Rechtsanwälte PartG mbB

(56) References cited:
- WO-A1-2006/131997
- US-A- 3 735 756
- US-A1- 2009 043 293
- US-A1- 2016 089 537

## Description

### BACKGROUND

### 1. Technical Field

The present disclosure relates to a beauty apparatus for use in a skin.

### 2. Description of the Related Art

Heretofore, a beauty apparatus that can beautify a skin, for example, by applying an ultrasonic wave to the skin has been known, for example, as disclosed in Japanese Patent No. 4416028.

Recently, a beauty apparatus has also been developed, which can further improve such a skin-beautifying effect by providing a skin with a plurality of different stimulations. In this case, the beauty apparatus must mount a plurality of drive units which correspond to the plurality of individual stimulations, resulting in apprehension about an increase of power consumption by that amount.

"Document US 2016/089537 A1 teaches a high-frequency cosmetic treatment apparatus capable of preventing local heating of a skin. The high-frequency cosmetic treatment apparatus includes a portable casing, side-by-side electrodes disposed at a tip portion of the casing with a predetermined interval provided therebetween and having treatment surfaces to be contacted with a skin of a user, and a first power supply disposed in the casing to supply a high-frequency current to the pair of side-by-side electrodes".

### SUMMARY

The present disclosure provides a beauty apparatus capable of generating the plurality of different stimulations while preventing the increase of the power consumption.

Specifically, a beauty apparatus according to an aspect of the present disclosure includes: a first stimulation provider that provides a skin with a first stimulation; a second stimulation provider that provides the skin with a second stimulation induced by a radio frequency (RF) and different in type from the first stimulation; and a power supply unit that supplies the first stimulation provider and the second stimulation provider with power from a power supply.

The power supply unit is configured to be capable of alternately supplying the first stimulation provider and the second stimulation provider individually with the power from the power supply; the beauty apparatus further comprising a first heater and a second heater which heat the skin from a surface of the skin, wherein the first heater and the second heater are driven at a time of preheating, and either one of the first heater and the second heater is driven after the preheating.

According to the present disclosure, a beauty apparatus can be provided which is capable of generating the plurality of different stimulations while preventing the increase of the power consumption.

### BRIEF DESCRIPTION OF THE DRAWINGS

FIG. 1 is a plan view illustrating a schematic configuration of a beauty apparatus according to an exemplary embodiment of the present disclosure;
FIG. 2 is a sectional view illustrating the schematic configuration of the beauty apparatus according to the exemplary embodiment of the present disclosure;
FIG. 3 is a plan view illustrating a schematic configuration of a head according to the exemplary embodiment of the present disclosure;
FIG. 4 is a sectional view illustrating the schematic configuration of the head according to the exemplary embodiment of the present disclosure;
FIG. 5 is a perspective view illustrating a schematic configuration of a second holder and a second stimulation provider according to the exemplary embodiment of the present disclosure;
FIG. 6 is a perspective view illustrating a state where a part of the second holder according to the exemplary embodiment of the present disclosure is detached;
FIG. 7 is a perspective view illustrating a schematic configuration of a terminal according to the exemplary embodiment of the present disclosure;
FIG. 8 is a perspective view illustrating a schematic configuration of a conductive plate according to the exemplary embodiment of the present disclosure;
FIG. 9 is a block diagram illustrating a control configuration of the beauty apparatus according to the exemplary embodiment of the present disclosure;
FIG. 10 is a sectional view illustrating a state where a protrusion of a probe head according to the exemplary embodiment of the present disclosure is pressed against a skin;
FIG. 11 is a timing chart illustrating operation timing of each portion in an alternate mode according to the exemplary embodiment of the present disclosure; and
FIG. 12 is a timing chart illustrating operation timing of each portion in the alternate mode according to the exemplary embodiment of the present disclosure.

### DETAILED DESCRIPTION

Hereinafter, a beauty apparatus according to an exemplary embodiment of the present disclosure will be described in detail with reference to the drawings. The exemplary embodiment described below is a preferred specific example of the present disclosure. Therefore, numeric values, shapes, materials, constituents, dispositions and connection modes of the constituents, and the like, which are shown in the following exemplary embodiment, are merely examples, and are not intended to limit the present disclosure. Accordingly, among the constituents in the following exemplary embodiment, constituents which are not recited in the independent claim for the most generic concept of the present disclosure are described as arbitrary constituents.

The drawings are also schematic views, and are not always exactly illustrated. In the respective drawings, identical components are denoted by identical reference symbols.

### [Beauty apparatus]

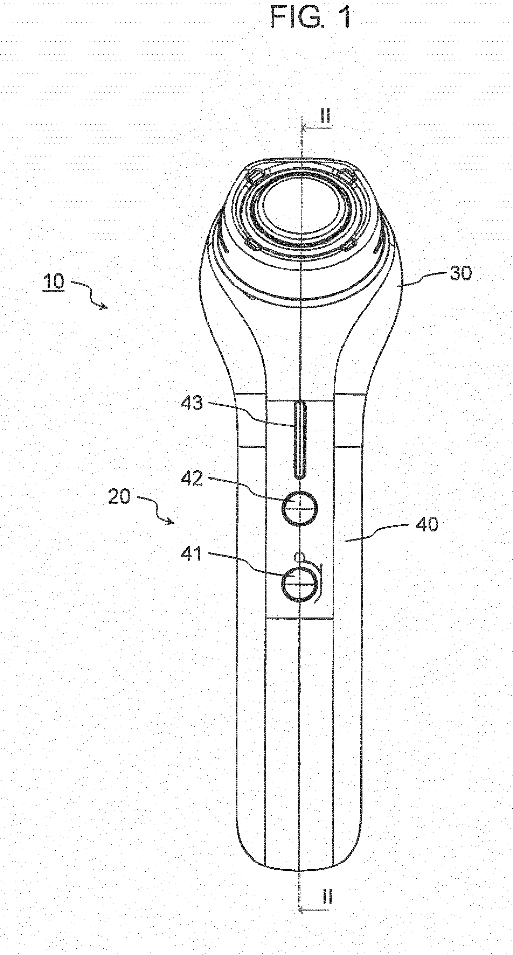

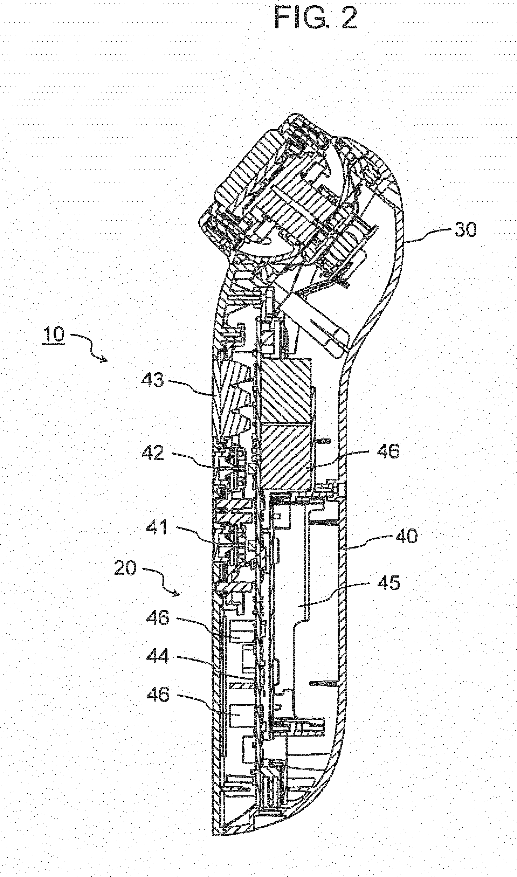

First, beauty apparatus 10 according to the exemplary embodiment of the present disclosure will be described. FIG. 1 is a plan view illustrating a schematic configuration of beauty apparatus 10 according to the exemplary embodiment of the present disclosure. FIG. 2 is a sectional view illustrating the schematic configuration of beauty apparatus 10 according to the exemplary embodiment of the present disclosure. Specifically, FIG. 2 is a sectional view illustrating a section including section line II-II in FIG. 1.

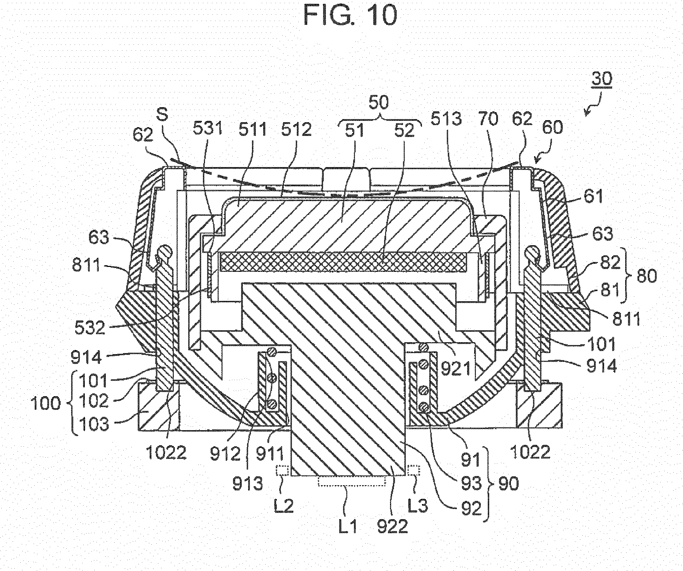

As illustrated in FIGS. 1 and 2, beauty apparatus 10 includes long housing 20 as an exterior body. One end of housing 20 serves as head 30 that provides stimulations to skin S (see FIG. 10). A part other than head 30 in housing 20 is gripper 40 gripped by a user. Gripper 40 is formed narrower than head 30. Gripper 40 has power button 41 for switching on and off power, selection button 42 for selecting an operation mode of beauty apparatus 10, and lighting portion 43 which is turned on when the power is switched on and is turned off when the power is switched off.

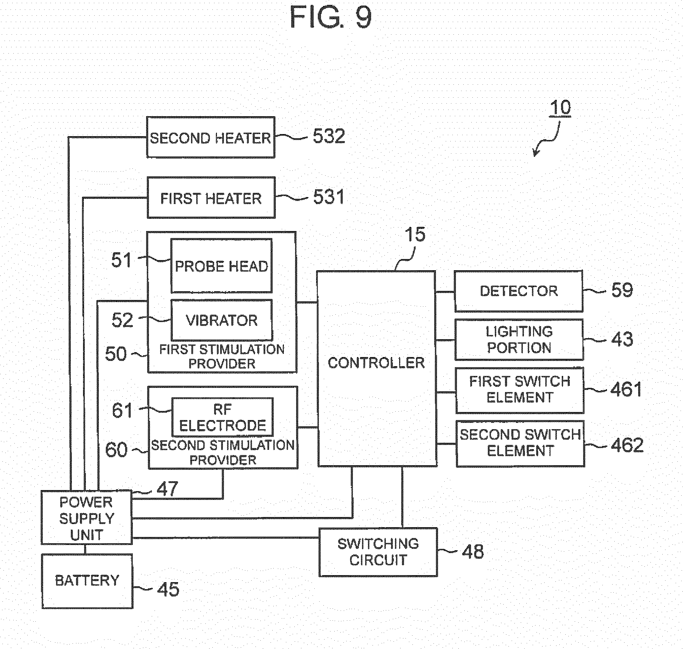

As illustrated in FIG. 2, circuit board 44 and battery 45 are accommodated inside gripper 40. A plurality of circuit components 46 for driving drive units of head 30 are mounted on circuit board 44. The plurality of circuit components 46 include a microcomputer. The microcomputer is controller 15 (see FIG. 9) for controlling the drive units of beauty apparatus 10.

The plurality of circuit components 46 further include first and second switch elements 461 and 462 (see FIG. 9). First switch element 461 is used for turning on and off power. Pressing down of power button 41 outputs, to controller 15, a control signal for switching on and off power supply from battery 45. Second switch element 462 is used for selecting the operation mode. Pressing down of selection button 42 outputs, to controller 15, a control signal corresponding to each operation mode.

The plurality of circuit components 46 further include: power supply unit 47 for supplying the power from battery 45 individually to first and second stimulation providers 50 and 60 to be described later; and switching circuit 48 for outputting a signal to power supply unit 47 (see FIG. 9). Power supply unit 47 and switching circuit 48 will be described later.

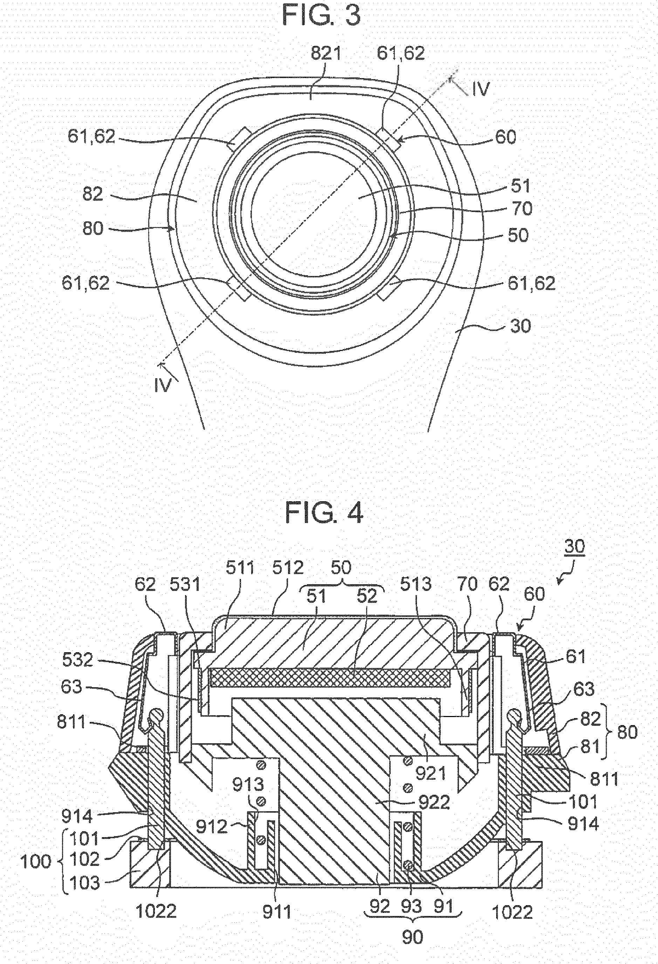

FIG. 3 is a plan view illustrating a schematic configuration of head 30 according to the exemplary embodiment of the present disclosure. FIG. 4 is a sectional view illustrating the schematic configuration of head 30 according to the exemplary embodiment of the present disclosure. Specifically, FIG. 4 is a sectional view illustrating a section including section line IV-IV in FIG. 3.

As illustrated in FIGS. 3 and 4, head 30 includes first stimulation provider 50, second stimulation provider 60, first holder 70, second holder 80, retractable mechanism 90, and terminal 100. First holder 70 and second holder 80 hold first stimulation provider 50 and second stimulation provider 60 such that second stimulation provider 60 is disposed close to first stimulation provider 50. In this manner, first and second stimulation providers 50 and 60 can simultaneously come into contact with skin S of the user.

First stimulation provider 50 provides skin S with a first stimulation. The first stimulation includes an ultrasonic wave vibration stimulation, an electrical stimulation (for example, electrical muscle stimulation: EMS), a thermal stimulation, and a light stimulation. In the present exemplary embodiment, the ultrasonic wave vibration stimulation will be described as an example of the first stimulation.

First stimulation provider 50 includes probe head 51 and vibrator 52 which vibrates probe head 51. Probe head 51 is a metal body which comes into contact with skin S and transmits vibration generated by vibrator 52 to skin S. In probe head 51, a portion exposed from head 30 protrudes in a disc shape. A surface of protrusion 511 serves as oxide film 512. For example, in the present exemplary embodiment, after probe head 51 is formed of aluminum, protrusion 511 is subjected to anodization, whereby oxide film 512 formed of aluminum oxide is generated. A surface of probe head 51 may entirely serve as oxide film 512.

Circularly erected peripheral wall 513 is formed on a side opposite to protrusion 511 in probe head 51. On an outer peripheral surface of peripheral wall 513, sheet-shaped first and second heaters 531 and 532 are disposed to be arrayed in an axial direction of probe head 51. First and second heaters 531 and 532 are attached to an entire outer peripheral surface of peripheral wall 513. First heater 531 is a heater dedicated to preheating, and preheats probe head 51 before treatment. Second heater 532 heats skin S via probe head 51 by heating probe head 51. First and second heaters 531 and 532 are electrically connected to power supply unit 47. An output of first heater 531 is set larger than an output of second heater 532. Specifically, the output of first heater 531 is 4.2 W, and the output of the second heater 532 is 1.8 W. In this manner, overheating for skin S can be prevented at a time of the treatment while reducing a time taken for the preheating.

Vibrator 52 is a vibrator which vibrates probe head 51 by vibrating in a frequency band of the ultrasonic wave and provides the ultrasonic wave vibration stimulation to skin S in contact with probe head 51. Specifically, vibrator 52 is a piezoelectric element, and is fixed to probe head 51 inside a region surrounded by peripheral wall 513. A vibration frequency of vibrator 52 is set to approximately 1 MHz.

First holder 70 is a resin member which holds first stimulation provider 50. Specifically, first holder 70 is formed in a substantially cylindrical shape for example. First holder 70 is attached to an outer peripheral edge of protrusion 511 of probe head 51 such that protrusion 511 protrudes outward from one end of first holder 70 itself. First holder 70 is connected to retractable mechanism 90.

Retractable mechanism 90 is a mechanism for retracting first stimulation provider 50 to second stimulation provider 60 by retracting first holder 70 to head 30. Specifically, retractable mechanism 90 includes base 91, connector 92, and elastic member 93.

Base 91 is a resin member configuring a part of head 30, and is formed in a substantial cup shape. Head 30 is configured such that connector 92 moves relatively to base 91. Specifically, through hole 911 is formed in a center of base 91. Tubular portion 912 concentric with through hole 911 is formed around through hole 911. Tubular portion 912 has accommodation groove 913 which accommodates elastic member 93. Accommodation groove 913 is formed concentrically with through hole 911. A plurality of attachment holes 914 are formed on an outer peripheral portion of base 91. A plurality of electrode pins 101 included in terminal 100 are individually attached to the plurality of attachment holes 914.

Connector 92 is a resin member connected to first holder 70. Connector 92 includes: fitting portion 921 fitted to first holder 70; and shaft 922 protruding from fitting portion 921 and inserted into through hole 911 of base 91.

Elastic member 93 applies an urging force, which goes outward of head 30, to connector 92 when elastically deformed. Specifically, elastic member 93 is a coil spring for example. One end of elastic member 93 is fixed into accommodation groove 913, and the other end is fixed to fitting portion 921 of connector 92. That is, elastic member 93 is configured to be extended and contracted by being elastically deformed and elastically restored in a state where elastic member 93 is accommodated in accommodation groove 913. Elastic member 93 may be a spring other than the coil spring, or may be other elastic bodies such as rubber. A retracting operation performed by retractable mechanism 90 will be described later.

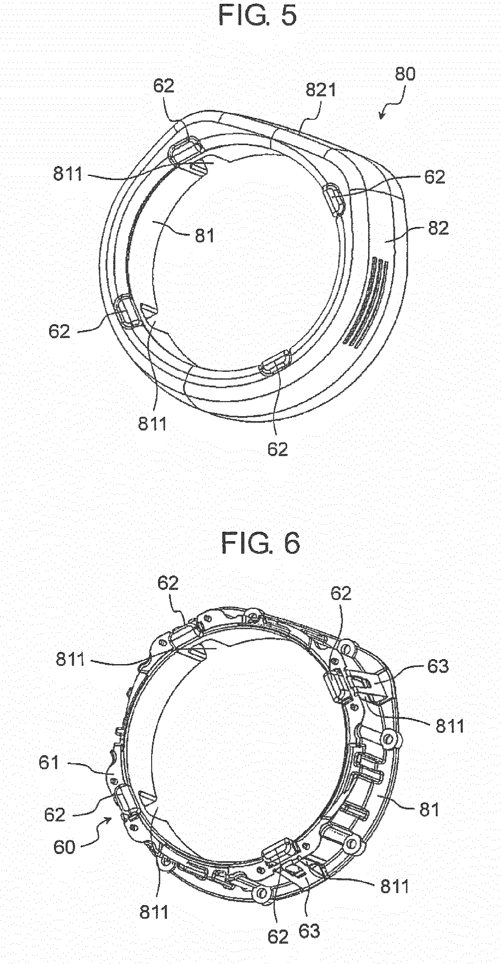

FIG. 5 is a perspective view illustrating a schematic configuration of second holder 80 and second stimulation provider 60 according to the exemplary embodiment of the present disclosure. FIG. 6 is a perspective view illustrating a state where a part of second holder 80 according to the exemplary embodiment of the present disclosure is detached.

As illustrated in FIGS. 4 to 6, second stimulation provider 60 is integrally assembled with second holder 80, and second holder 80 is detachable from first holder 70 in a state where second stimulation provider 60 is integrally assembled with second holder 80. Specifically, second holder 80 is detachable from base 91 for first holder 70.

Second stimulation provider 60 provides skin S with a stimulation induced by a radio frequency (hereinafter, abbreviated as an "RF"). Second stimulation provider 60 provides skin S with a second stimulation which is different in type from the first stimulation. Examples of the second stimulation include a stimulation for heating a deep portion of skin S by generating an electric field or a magnetic field in a range from a surface of skin S to an inside of skin S using the RF. In the present exemplary embodiment, as an example of the second stimulation, a stimulation induced by the electric field generated by the RF will be described.

Specifically, second stimulation provider 60 includes RF electrode 61 formed in an annular shape. RF electrode 61 is formed of conductive metal. For example, in the present exemplary embodiment, RF electrode 61 is formed of titanium. RF electrode 61 has a plurality of projections 62 protruding from second holder 80. The plurality of projections 62 is arranged at an equal interval in a circumferential direction. In the present exemplary embodiment, as illustrated in FIG. 3, a case where four projections 62 are provided is described as an example. Herein, in each of the projections 62, a shape of a portion exposed from second holder 80 is rectangular when viewed from the above. However, the shape and the number of projections 62 may be arbitrary.

As illustrated in FIGS. 4 to 6, locking pieces 63 extending in a direction opposite to a projecting direction of projections 62 are formed on portions of RF electrode 61, the portions facing projections 62. Therefore, RF electrode 61 includes locking pieces 63 of which number is the same as the number of the plurality of projections 62. Tip ends of locking pieces 63 are formed in a bent plate spring shape. As illustrated in FIG. 4, electrode pins 101 of terminal 100 are locked to the tip ends of locking pieces 63.

In a case where second stimulation provider 60 provides skin S with the stimulation induced by the electric field generated by the RF, the other RF electrode different in polarity from RF electrode 61 is required. In the present exemplary embodiment, probe head 51 of first stimulation provider 50 also serves as the other RF electrode. In this manner, as a whole, head 30 includes a pair of RF electrodes (RF electrode 61 and probe head 51) different in polarity from each other.

Here, the RF just needs to be an electromagnetic wave in a frequency band of 10 kHz or more to 100 GHz or less. From a viewpoint of a beautifying effect, it is more desirable that the RF be an electromagnetic wave or a current in a frequency band of 0.3 MHz or more to 50 MHz or less. The present exemplary embodiment employs, as the RF, a current at approximately 1 MHz, which is suitable for aluminum-made probe head 51 provided with oxide film 512. That is, the vibration frequency of vibrator 52 and the frequency of the current for generating the second stimulation coincide with each other.

Second holder 80 is a member freely detachable from first holder 70 and holding second stimulation provider 60. Specifically, as a whole, second holder 80 has an annular outer shape, and first stimulation provider 50 is disposed inside an opening portion of second holder 80. Second holder 80 includes frame 81 and cover 82. Frame 81 is a resin member formed annularly. Frame 81 holds RF electrode 61 of second stimulation provider 60. On frame 81, guide routes 811 are formed. Guide routes 811 guide electrode pins 101 of terminal 100 to locking pieces 63 of RF electrode 61. Cover 82 is composed of, for example, a resin member formed annularly. Cover 82 is attached to frame 81, thereby covering the other portion of RF electrode 61 in a state where projections 62 of RF electrode 61 are individually exposed. As illustrated in FIG. 3, a part of an edge of cover 82 is formed in a flat shape. A part of the edge of cover 82 formed in a flat shape will be referred to as flat portion 821. Flat portion 821 is located in a tip end portion of housing 20 (see FIG. 1), and extends in a direction perpendicular to a longitudinal direction of housing 20. For example, in a case where an opening portion of cover 82 is circular, flat portion 821 is formed in a flat shape substantially parallel to a tangent line of the opening portion. With such a configuration, flat portion 821 in cover 82 is disposed closer to first stimulation provider 50 and second stimulation provider 60 than the other portion in cover 82. Specifically, as illustrated in FIG. 3, entire flat portion 821 faces probe head 51 of first stimulation provider 50. Two projections 62 of second stimulation provider 60 face both ends of flat portion 821.

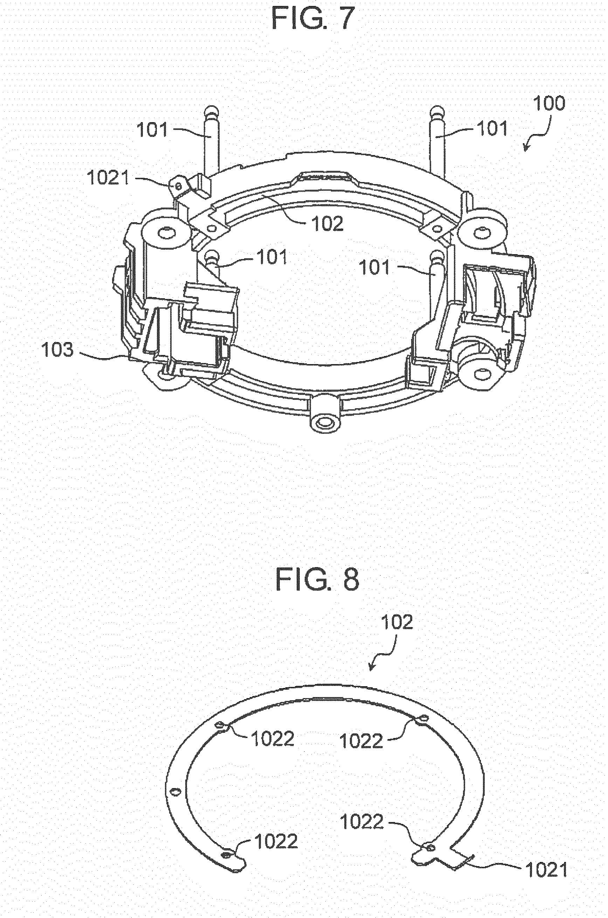

FIG. 7 is a perspective view illustrating a schematic configuration of terminal 100 according to the exemplary embodiment of the present disclosure.

As illustrated in FIGS. 4 and 7, terminal 100 is a region for supplying power to second stimulation provider 60 when second stimulation provider 60 is electrically connected. Specifically, terminal 100 includes the plurality of electrode pins 101, conductive plate 102, and bracket 103.

Each of electrode pins 101 is formed in a columnar shape, and has a tip end formed in a substantially spherical shape. Electrode pins 101 cause their tip ends to protrude from attachment holes 914 of base 91. Locking pieces 63 of RF electrode 61 are locked with the tip ends of electrode pin 101, whereby second holder 80 is attached to first holder 70 (see FIG. 4). In this state, RF electrode 61 and electrode pins 101 are electrically connected to each other.

Meanwhile, if second holder 80 is pulled in a direction away from first holder 70, then the tip ends of electrode pins 101 and locking pieces 63 are unlocked from each other, and second holder 80 is detached from first holder 70 (see FIG. 5). In this state, RF electrode 61 and electrode pins 101 are not electrically connected to each other.

FIG. 8 is a perspective view illustrating a schematic configuration of conductive plate 102 according to the exemplary embodiment of the present disclosure. FIG. 8 is a perspective view of conductive plate 102 when viewed in a direction different from that in FIG. 7. As illustrated in FIG. 8, conductive plate 102 is a conductive metal plate formed in a substantially C shape. Tongue portion 1021 protruding outward is formed in one end of conductive plate 102. Tongue portion 1021 is electrically connected to power supplying circuit component 46 via a wire (not illustrated). A C-shaped portion of conductive plate 102 has a plurality of connection holes 1022 to which the plurality of electrode pins 101 is individually connected. When electrode pins 101 are connected to connection holes 1022, conductive plate 102 and electrode pins 101 are electrically connected to each other. As mentioned above, the plurality of electrode pins 101 is electrically connected to RF electrode 61 of second stimulation provider 60. Accordingly, conductive plate 102 and RF electrode 61 are electrically connected to each other via electrode pins 101. That is, terminal 100 and second stimulation provider 60 are electrically connected to each other at a plurality of spots. Accordingly, even if one of electrode pins 101 is broken and becomes incapable of electrically connecting terminal 100 and second stimulation provider 60 to each other, other electrode pins 101 can continue to electrically connect terminal 100 and second stimulation provider 60 to each other.

As illustrated in FIG. 7, bracket 103 is a member which holds the plurality of electrode pins 101 and conductive plate 102. Bracket 103 is formed annularly, and a part of retractable mechanism 90 is disposed inside an opening portion of bracket 103 (see FIG. 4). Bracket 103 is fixed into housing 20.

### [Control configuration]

Next, a control configuration of beauty apparatus 10 will be described. FIG. 9 is a block diagram illustrating the control configuration of beauty apparatus 10 according to the exemplary embodiment of the present disclosure.

Controller 15 of beauty apparatus 10 is, for example, a microcomputer including a timer. As illustrated in FIG. 9, to controller 15, there are electrically connected first stimulation provider 50, second stimulation provider 60, first switch element 461, second switch element 462, lighting portion 43, power supply unit 47, switching circuit 48, and detector 59. Based on the control signals output from first and second switch elements 461 and 462 and on a detection result of detector 59, controller 15 controls first stimulation provider 50, second stimulation provider 60, lighting portion 43, battery 45, power supply unit 47, and switching circuit 48.

Power supply unit 47 is a power supply circuit which supplies the power from battery 45 as a power supply to each of first stimulation provider 50, second stimulation provider 60, first heater 531, and second heater 532. Specifically, power supply unit 47 is electrically connected to first stimulation provider 50, second stimulation provider 60, first heater 531, second heater 532, and battery 45. Based on the control of controller 15, power supply unit 47 supplies the power from battery 45 to each of first stimulation provider 50, second stimulation provider 60, first heater 531, and second heater 532.

For example, upon receiving, from controller 15, a first instruction signal for providing the first stimulation, power supply unit 47 supplies the power to vibrator 52 of first stimulation provider 50. Meanwhile, upon receiving, from controller 15, a second instruction signal for providing the second stimulation, power supply unit 47 supplies the power to probe head 51 of first stimulation provider 50 and RF electrode 61 of second stimulation provider 60. This is because RF electrode 61 and probe head 51 are used as a pair of the RF electrodes for providing the second stimulation.

Herein, controller 15 outputs the instruction signal to power supply unit 47 based on the control signal input from second switch element 462, that is, based on a control signal corresponding to the operation mode selected in such a manner that the user operates selection button 42.

The operation mode includes: an alternate mode in which first stimulation provider 50 and second stimulation provider 60 operate alternately; and a continuous mode in which both of first stimulation provider 50 and second stimulation provider 60 operate continuously.

In the alternate mode, controller 15 outputs the first instruction signal and the second instruction signal to power supply unit 47 while alternately repeating the first instruction signal and the second instruction signal. In the present exemplary embodiment, an output period of the first instruction signal and an output period of the second instruction signal are equalized with each other. Specifically, the output period is set to 1 second for example. The output period of each of the first instruction signal and the second instruction signal just needs to be 0.1 seconds or more and 2 seconds or less.

In the alternate mode, power supply unit 47 repeats the supply of the power to vibrator 52 and stop of the supply every 1 second. At this time, power supply unit 47 stops supplying the power to probe head 51 and RF electrode 61 when the power is supplied to vibrator 52, and supplies the power to probe head 51 and RF electrode 61 when the supply of the power to vibrator 52 is stopped. In this manner, the first stimulation and the second stimulation will be alternately provided to skin S.

As described above, in the alternate mode, the power is alternately supplied to vibrator 52 for providing the first stimulation and probe head 51 and RF electrode 61 for providing the second stimulation. Accordingly, power consumption can be reduced.

Moreover, in the alternate mode, each of the first stimulation and the second stimulation is provided intermittently to skin S, and stop periods of both of the stimulations depend on the output periods of the first and second instruction signals. The output periods are 0.1 seconds or more and 2 seconds or less as mentioned above, and therefore, the stop periods are substantially the same as the output periods. Each of the first and second stimulations functions to activate skin S. Even when the application of stimulations is stopped, a degree of activating skin S gradually decreases. However, it takes a certain time to zero the degree of activating skin S. If the stop periods are as relatively short as 0.1 seconds or more and 2 seconds or less, the degree of activating skin S is not zeroed. Even if each of the first stimulation and the second stimulation is provided intermittently to skin S, a certain skin-beautifying effect can be obtained.

In the present exemplary embodiment, a period which remains within a range of 0.1 seconds or more and 2 seconds or less is mentioned as an example of the stop periods (output periods). The stop periods just need to be periods while the degree of activating skin S by each of the first stimulation and the second stimulation does not decrease to a predetermined value.

In the continuous mode, controller 15 continuously outputs the first instruction signal and the second instruction signal to power supply unit 47. In the continuous mode, power supply unit 47 performs the supply of the power to vibrator 52 and the supply of the power to probe head 51 and RF electrode 61 simultaneously and continuously.

As described above, in the continuous mode, each of the first stimulation and the second stimulation is continuously provided to skin S. Accordingly, in comparison with the alternate mode, it is possible to exert a higher skin-beautifying effect.

Moreover, upon receiving, from controller 15, a first heating signal for driving first heater 531, power supply unit 47 supplies power to first heater 531. Likewise, upon receiving, from controller 15, a second heating signal for driving second heater 532, power supply unit 47 supplies power to second heater 532.

Switching circuit 48 is a circuit which monitors the control of controller 15 for power supply unit 47 and switches power supply unit 47 to a stop state when abnormality occurs in the control. Switching circuit 48 does not operate when controller 15 is normally driven, but operates when controller 15 becomes abnormal, for example, runs away out of control. Specifically, switching circuit 48 monitors whether the first instruction signal and the second instruction signal, which are output from controller 15, are output periodically in the alternate mode, thereby determining whether controller 15 is abnormal. Switching circuit 48 has a circuit configuration of determining that the control of controller 15 is abnormal when the output period of the first instruction signal or the second instruction signal exceeds a certain time. For example, a timer circuit is usable as switching circuit 48. The timer circuit outputs a stop signal to power supply unit 47 when the output period of either one of the first instruction signal and the second instruction signal exceeds the certain time, that is, when the control of controller 15 is abnormal. The timer circuit resets the timer when the instruction signal is switched to the other signal within the certain time, that is, when the control of controller 15 is normal. In this manner, power supply unit 47 is stopped when the control of controller 15 is abnormal, and the operation of power supply unit 47 is continued when the control of controller 15 is normal. Herein, the certain time is defined to be a time larger than the preset output period (1 second in the present exemplary embodiment).

Upon receiving the control signal from first switch element 461 in such a manner that power button 41 is operated when the power supply is off, controller 15 controls power supply unit 47 to supply the respective portions with the power from battery 45. Meanwhile, upon receiving the control signal from first switch element 461 in such a manner that power button 41 is operated when the power supply is on, controller 15 controls power supply unit 47 to stop supplying the respective portions with the power from battery 45. Controller 15 turns on lighting portion 43 when the power supply is on, and turns off lighting portion 43 when the power supply is off.

Upon receiving the control signal corresponding to each of the operation modes from second switch element 462 in such a manner that selection button 42 is operated when the power supply is on, controller 15 controls power supply unit 47 in response to the operation mode concerned. In any of the operation modes, controller 15 controls power supply unit 47 based on the detection result of detector 59.

Detector 59 is a sensor which detects the contact of skin S with first stimulation provider 50 and second stimulation provider 60. Detector 59 can employ any of a mechanical system, an optical system and an electrical system.

First, mechanical detector 59 will be described.

FIG. 10 is a sectional view illustrating a state where protrusion 511 of probe head 51 according to the exemplary embodiment of the present disclosure is pressed against skin S. Specifically, FIG. 10 corresponds to FIG. 4. In FIG. 10, an outline of skin S is illustrated by a two-dot chain line.

When protrusion 511 of probe head 51 is pressed against skin S in a state illustrated in FIG. 4, probe head 51 descends from head 30, and elastic member 93 is contracted as illustrated in FIG. 10. When skin S is brought into contact with both of probe head 51 and projections 62 of RF electrode 61, probe head 51 stops descending. The switch element as detector 59 is disposed at a spot against which any one of first holder 70, first stimulation provider 50 and retractable mechanism 90 abuts when probe head 51 stops ascending. When detector 59 receives the abutment, a detection signal is output from detector 59 to controller 15. When detector 59 does not receive the abutment, no detection signal is output from detector 59. That is, a state where detector 59 outputs the detection signal indicates that skin S is in contact with both of probe head 51 and projections 62 of RF electrode 61.

For example, detector 59 which is mechanical is disposed on a spot (see a broken line L1 in FIG. 10) against which a tip end surface of shaft 922 of connector 92 abuts when skin S is brought into contact with both of probe head 51 and projections 62 of RF electrode 61.

Next, optical detector 59 will be described.

Optical detector 59 includes a light emitter and a light receiver. Optical detector 59 outputs different signals depending on whether a current state is switched between a light receiving state where the light receiver receives light from the light emitter and a non-light receiving state where the light receiver does not receive the light from the light emitter. In the present exemplary embodiment, such a signal output in the non-light receiving state is defined as the detection signal. The light emitter and the light receiver are disposed at a position where the light from the light emitter is not blocked in the state illustrated in FIG. 4 and where the light from the light emitter is blocked in the state illustrated in FIG. 10. In this manner, when skin S is brought into contact with both of probe head 51 and projections 62 of RF electrode 61 and probe head 51 stops descending, then the light from the light emitter is blocked to bring the non-light receiving state, and the detection signal is output from detector 59 to controller 15.

Examples of arrangement of detector 59 include that detector 59 which is optical is disposed on spots (see broken lines L2 and L3 in FIG. 10) where shaft 922 of connector 92 enters between the light emitter and the light receiver and blocks light when skin S is brought into contact with both of probe head 51 and projections 62 of RF electrode 61.

Next, electrical detector 59 will be described.

Electrical detector 59 outputs the detection signal to controller 15 in a case where the RF electrodes (probe head 51 and RF electrode 61) making a pair are electrically connected to each other via skin S when the RF electrodes are brought into contact with skin S. Specifically, detector 59 measures a current value between the pair of RF electrodes. When the current value exceeds a threshold, detector 59 determines that the pair of RF electrodes are electrically connected to each other via skin S, and outputs the detection signal to controller 15.

Based on a result of the detection by detector 59, controller 15 controls power supply unit 47. Specifically, when the detection signal output from detector 59 is not present, that is, when detector 59 does not detect the contact of skin S, controller 15 does not output the first instruction signal and the second instruction signal to power supply unit 47. That is, first stimulation provider 50 and second stimulation provider 60 do not operate.

Meanwhile, when the detection signal output from detector 59 is present, that is, when detector 59 detects the contact of skin S, controller 15 outputs the first instruction signal and the second instruction signal to power supply unit 47 so as to correspond to each of the operation modes. In this manner, first stimulation provider 50 and second stimulation provider 60 operate in response to each of the operation modes.

When the detection signal output from detector 59 is present, controller 15 blinks lighting portion 43. When the detection signal output from detector 59 is gone, controller 15 lights lighting portion 43. As described above, a lighting condition of lighting portion 43 changes depending on whether the detection signal is present. Accordingly, the user can be notified that skin S is in contact with both of probe head 51 and projections 62 of RF electrode 61.

### [Operations]

Next, operations of beauty apparatus 10 will be described.

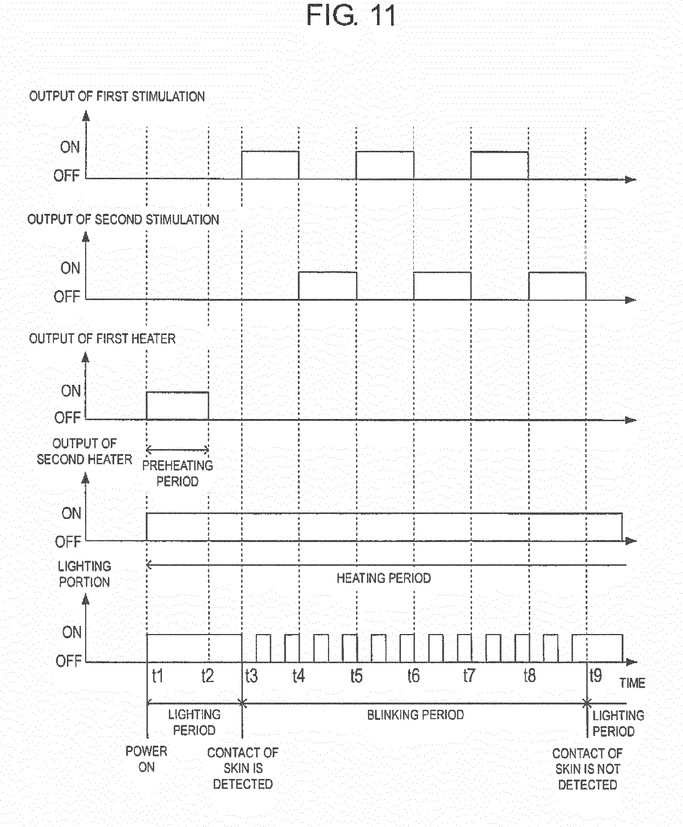

First, operations in the alternate mode will be described. FIG. 11 is a timing chart illustrating operation timing of each portion in the alternate mode according to the exemplary embodiment of the present disclosure.

First, the user applies moisture-containing cosmetics or treatment gel to at least one of skin S to be treated and probe head 51 of beauty apparatus 10. Thereafter, if the user operates power button 41, the power supply becomes in an ON state in beauty apparatus 10. Timing when the power supply becomes in the ON state is indicated by time t1 in FIG. 11.

At this time, controller 15 controls and lights lighting portion 43. A lighting period here is a period from time t1 to time t3.

Moreover, at time t1, controller 15 outputs the first heating signal and the second heating signal to power supply unit 47, thereby supplying first and second heaters 531 and 532 with power. In this manner, first and second heaters 531 and 532 are driven to preheat probe head 51.

Thereafter, when a predetermined preheating period elapses at time t2, controller 15 stops outputting the first heating signal to power supply unit 47. In this manner, first heater 531 is not supplied with the power, and stops. Meanwhile, second heater 532 continues to be supplied with the power. Accordingly, probe head 51 continues to be heated at a constant temperature.

Moreover, during a period from time t1 to time t3, as illustrated in FIG. 4, elastic member 93 of retractable mechanism 90 is extended, and protrusion 511 of probe head 51 protrudes from head 30.

Subsequently, when the user operates selection button 42 to select the alternate mode, controller 15 prepares the control in the alternate mode. Thereafter, the user then presses protrusion 511 of probe head 51 against skin S. When probe head 51 descends from head 30, and skin S is brought into contact with protrusion 511 of probe head 51 and projections 62 of RF electrode 61 as illustrated in FIG. 10, then the detection signal indicating the contact of skin S is output from detector 59 to controller 15. Timing when the detection signal is output to controller 15 is indicated by time t3 in FIG. 11.

Upon receiving the detection signal, controller 15 outputs the first instruction signal and the second instruction signal to power supply unit 47 while alternately repeating the first instruction signal and the second instruction signal. In this manner, in the alternate mode, the power is alternately supplied to vibrator 52 for providing the first stimulation and probe head 51 and RF electrode 61 for providing the second stimulation. Hence, the first stimulation and the second stimulation are alternately provided to skin S. Specifically, in FIG. 11, the first stimulation is output during a period of time t3 to time t4, a period of time t5 to time t6, and a period of time t7 to time t8. Meanwhile, the second stimulation is output during a period of time t4 to time t5, a period of time t6 to time t7, and a period of time t8 to time t9. During a period of time t3 to time t9, three stimulations to be described below are provided to skin S.

The respective stimulations will be specifically described.

First, the first stimulation is the ultrasonic wave vibration induced by vibrator 52. The ultrasonic wave vibration generated by vibrator 52 is provided to skin S via probe head 51. The ultrasonic wave vibration is provided to skin S, thereby allowing moisture distribution inside skin S to be uniform. Accordingly, tension can be provided to skin S.

Next, the second stimulation is the RF induced by the pair of RF electrodes (probe head 51 and RF electrode 61). When both of probe head 51 and RF electrode 61 come into contact with skin S in a state of being applied with a voltage in the frequency band where the RF is generated, an electric field is generated at a specific region of skin S, and Joule heat generated by a current is generated in the region. When the specific region of skin S is heated as described above, skin S can be activated by a promoted blood flow and an action of gene expression.

The third stimulation is heat induced by second heater 532. When second heater 532 is heated, probe head 51 in contact with second heater 532 is also heated. In this manner, the heat is transferred from probe head 51 to skin S, and skin S is warmed. When skin S is warmed as described above, the current becomes easy to flow in skin S. Accordingly, it becomes easy to generate the electric field, which is induced by the RF, in skin S, and the current becomes easy to flow. Hence, it becomes easier to warm the deep portion of skin S, and a heating effect achieved using the RF can be improved.

Note that, upon receiving the detection signal, controller 15 controls and blinks lighting portion 43. The period of time t3 to time t9 in FIG. 11 is a blinking period. The user sees the blink of lighting portion 43 directly or via a mirror, and can thereby know that skin S comes into contact with protrusion 511 of probe head 51 and projections 62 of RF electrode 61 and is provided with the respective stimulations.

In a state where probe head 51 is pressed against skin S, the user moves beauty apparatus 10 such that probe head 51 slides on skin S. In this case, if beauty apparatus 10 is moved in a direction where flat portion 821 moves forward, the user can obtain a more lifted feeling.

Thereafter, when the contact of skin S with probe head 51 is released from the state illustrated in FIG. 10 by the operation of the user, elastic member 93 is elastically restored to extend as illustrated in FIG. 4, and probe head 51 returns to an original position of itself. In this state, probe head 51 protrudes from head 30, and accordingly, detector 59 does not output the detection signal. Timing when the input of the detection signal to controller 15 is stopped is indicated by time t9 in FIG. 11. When the input of the detection signal from detector 59 is stopped as described above, controller 15 stops outputting the first and second instruction signals, thereby stopping the supply of the power from power supply unit 47 to vibrator 52, probe head 51, and RF electrode 61. In this manner, first stimulation provider 50 and second stimulation provider 60 stop.

At this time, when the input of the detection signal to controller 15 is stopped, controller 15 controls and lights lighting portion 43. A lighting period here is a period on and after time t9 in FIG. 11.

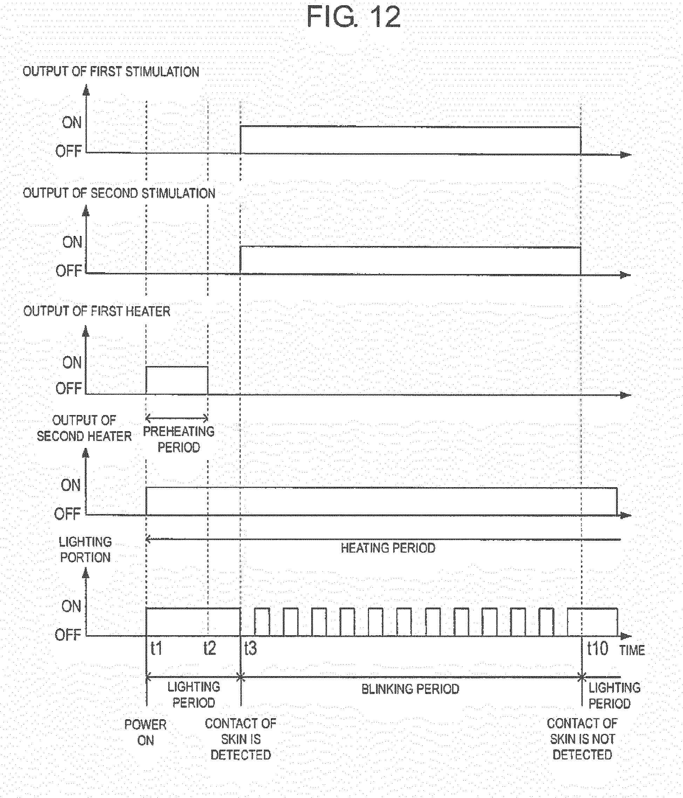

Next, operations in the continuous mode will be described. FIG. 12 is a timing chart illustrating operation timing of each portion in the continuous mode according to the exemplary embodiment of the present disclosure.

Operations of first and second heaters 531 and 532, operations of lighting portion 43, and entire operations up to time t3 are the same as those in the alternate mode, and accordingly, a description of these will be omitted herein.

Upon receiving the detection signal from detector 59 at time t3, controller 15 continuously outputs the first and second instruction signals to power supply unit 47. In this manner, in the continuous mode, the power is continuously supplied to vibrator 52 for providing the first stimulation and probe head 51 and RF electrode 61 for providing the second stimulation. Hence, the first stimulation and the second stimulation are continuously provided to skin S. Specifically, in FIG. 12, the first to third stimulations are output during a period of time t3 to time t10.

When the contact of skin S with probe head 51 is released by the operation of the user, detector 59 does not output the detection signal. Timing when the input of the detection signal to controller 15 is stopped is indicated by time t10 in FIG. 12. When the input of the detection signal from detector 59 is stopped as described above, controller 15 stops outputting the first and second instruction signals, thereby stopping the supply of the power from power supply unit 47 to vibrator 52, probe head 51, and RF electrode 61. In this manner, first stimulation provider 50 and second stimulation provider 60 stop.

When abnormality occurs in controller 15 during the execution of the alternate mode, switching circuit 48 outputs the stop signal to power supply unit 47. In this manner, when controller 15 is abnormal, power supply unit 47 can be stopped, and skin S can be stopped being provided with the first and second stimulations.

### [Effects]

As described above, beauty apparatus 10 according to the exemplary embodiment of the present disclosure includes: first stimulation provider 50 that provides skin S with the first stimulation; second stimulation provider 60 that provides skin S with the second stimulation which is the stimulation induced by the RF and different in type from the first stimulation; and power supply unit 47 that supplies each of first stimulation provider 50 and second stimulation provider 60 with the power output from the power supply (battery 45). Power supply unit 47 is configured to be capable of alternately supplying first stimulation provider 50 and second stimulation provider 60 individually with the power from the power supply.

With this configuration, power supply unit 47 supplies the power alternately to first stimulation provider 50 and second stimulation provider 60. Accordingly, when either one of first stimulation provider 50 and second stimulation provider 60 operates, the other stops. That is, the power consumption can be reduced in comparison with the case where both of first stimulation provider 50 and second stimulation provider 60 are continuously operated at the same time.

Beauty apparatus 10 may further include: controller 15 that controls power supply unit 47; and switching circuit 48 that monitors the control of controller 15 for power supply unit 47 and switches power supply unit 47 to the stop state when the control becomes abnormal.

With this configuration, when abnormality in the control occurs, switching circuit 48 switches power supply unit 47 to the stop state, and accordingly, the first and second stimulations can be prevented from being continuously provided to skin S when abnormality occurs.

The first stimulation is the ultrasonic wave vibration, and the vibration frequency of the first stimulation and the frequency of the current for generating the second stimulation coincide with each other.

With this configuration, since the vibration frequency of the first stimulation and the frequency of the current for generating the second stimulation coincide with each other, the power supply can be made common with ease.

Beauty apparatus 10 may further include first heater 531 and second heater 532, which heat skin S from the surface. In this case, beauty apparatus 10 may be configured such that first and second heaters 531 and 532 are driven at the time of preheating, and that either one of first and second heaters 531 and 532 is driven after the preheating.

With this configuration, since first and second heaters 531 and 532 are driven at the time of preheating, it is possible to preheat probe head 51 to a target temperature in a relatively short time. Moreover, since either one of first and second heaters 531 and 532 is driven after the preheating, it is possible to reduce the power consumption at the heating after the preheating.

### [Others]

The beauty apparatus according to the present disclosure has been described above based on the exemplary embodiment. However, the present disclosure is not limited to the above exemplary embodiment.

For example, in the above exemplary embodiment, the case where probe head 51 also serving as one of the RF electrodes making a pair is formed of aluminum is described as an example. However, probe head 51 may be formed of other metal. The other metal includes stainless steel and titanium. Even in a case of using the other metal, it is preferable to produce the oxide film on the surface of the metal. In the above exemplary embodiment, as an example, the case is described, where probe head 51 of first stimulation provider 50 also serves as one of the RF electrodes making a pair. However, it is also possible to provide a dedicated member serving as the RF electrode. Even in a case of using the dedicated member serving as the RF electrode, it is preferable to produce the oxide film on the surface of the dedicated member. The production of the oxide film decreases the conductivity of the RF electrode. However, if the frequency band of the voltage supplied to the pair of RF electrodes is set to 0.3 MHz or more, certain conductivity can be ensured for the RF electrode even if the oxide film is present.

In the above exemplary embodiment, as an example, the case has been described, where the electric field is generated from the surface of skin S to the inside of skin S by the pair of RF electrodes, so that the current generates Joule heat. However, the second stimulation provider is also capable of providing skin S with a thermal action and a blood circulation promoting action by generating the magnetic field in skin S using one RF electrode. That is, the second stimulation in this case is induced by the magnetic field.

In the above exemplary embodiment, as an example, the case has been described, where either one (probe head 51) of the RF electrodes making a pair has oxide film 512, and other RF electrode 61 has no oxide film. However, the other RF electrode may have the oxide film. In this manner, corrosion resistance of the other RF electrode can be improved.

In the above exemplary embodiment, as an example, the case has been described, where retractable mechanism 90 allows first stimulation provider 50 to retract from second stimulation provider 60. However, the retractable mechanism may allow the second stimulation provider to retract from the first stimulation provider. In this case, it is desirable that the first stimulation provider be fixed to the housing. Even in this case, both of the first stimulation provider and the second stimulation provider can be reliably in close contact with skin S.

Moreover, in the above exemplary embodiment, the case where probe head 51 also serves as the RF electrode is described as an example. However, each of a pair of the RF electrodes may be a member dedicated for the RF electrode. In this case, the second stimulation provider includes the pair of RF electrodes. The pair of RF electrodes can generate the electric field inside skin S, so that the current can generate Joule heat. In this case, probe head 51 is usable as a dedicated member for the ultrasonic wave vibration.

In the above exemplary embodiment, lighting portion 43 for visual notification is described as an example of a notification unit. However, other notification units are usable as long as the notification units can issue notice to the user. The other notification units include, for example, a speaker for audio notification and a vibrator for tactile notification. A plurality of notification units different in type are usable in combination.

In the above exemplary embodiment, battery 45 is described as an example of the power supply. However, a commercial power supply connected to power supply unit 47 may be used as the power supply.

## Claims

1. A beauty apparatus (10) comprising:
a first stimulation provider (50) that provides a skin (S) with a first stimulation;
a second stimulation provider (60) that provides the skin with a second stimulation induced by a radio frequency (RF) and different in type from the first stimulation; and
a power supply unit (47) that supplies the first stimulation provider (50) and the second stimulation provider (60) with power from a power supply, wherein
the power supply unit (47) is configured to be capable of alternately supplying the first stimulation provider (50) and the second stimulation provider (60) individually with the power from the power supply;
the beauty apparatus (10) further comprising a first heater (531) and a second heater (532) which heat the skin (S) from a surface of the skin (S), wherein
the first heater (531) and the second heater (532) are driven at a time of preheating, and
either one of the first heater (531) and the second heater (532) is driven after the preheating.

2. The beauty apparatus (10) according to claim 1, further comprising:
a controller (15) that controls the power supply unit (47); and
a switching circuit (48) that monitors the control of the controller (15) for the power supply unit (47) and switches the power supply unit (47) to a stop state when abnormality occurs in the control.

3. The beauty apparatus (10) according to either one of claims 1 and 2, wherein
the first stimulation is an ultrasonic wave vibration, and
a vibration frequency of the first stimulation and a frequency of a current for generating the second stimulation coincide with each other.

## Patentansprüche

1. Kosmetikgerät (10), umfassend:
einen ersten Stimulationserzeuger (50), der eine Haut (S) mit einer ersten Stimulation versorgt;
einen zweiten Stimulationserzeuger (60), der die Haut mit einer zweiten Stimulation versorgt, die durch eine Hochfrequenz (HF) hervorgerufen wird und sich in ihrem Typ von der ersten Stimulation unterscheidet; und
eine Stromversorgungseinheit (47), die den ersten Stimulationserzeuger (50) und den zweiten Stimulationserzeuger (60) mit Strom von einer Stromversorgung versorgt, wobei
die Stromversorgungseinheit (47) dazu eingerichtet ist, abwechselnd den ersten Stimulationserzeuger (50) und den zweiten Stimulationserzeuger (60) einzeln mit dem Strom aus der Stromversorgung zu versorgen;
das Kosmetikgerät (10) weiterhin eine erste Heizeinrichtung (531) und eine zweite Heizeinrichtung (532) umfasst, die die Haut (S) von einer Oberfläche der Haut (S) erwärmen, wobei
die erste Heizeinrichtung (531) und die zweite Heizeinrichtung (532) zu einem Zeitpunkt des Vorheizens betrieben werden und
die erste Heizeinrichtung (531) oder die zweite Heizeinrichtung (532) nach dem Vorheizen betrieben werden.

2. Kosmetikgerät (10) nach Anspruch 1, weiterhin umfassend:
eine Steuereinheit (15), die die Stromversorgungseinheit (47) steuert; und
einen Umschaltschaltkreis (48), der die Steuerung der Steuereinheit (15) für die Stromversorgungseinheit (47) überwacht und die Stromversorgungseinheit (47) in einen Stoppzustand schaltet, wenn eine Abnormität bei der Steuerung auftritt.

3. Kosmetikgerät (10) nach einem der Ansprüche 1 und 2, bei dem die erste Stimulation eine Ultraschallwellenschwingung ist, und
eine Schwingungsfrequenz der ersten Stimulation und eine Frequenz eines Stroms zum Erzeugen der zweiten Stimulation miteinander übereinstimmen.

## Revendications

1. Appareil de soin de beauté (10) comprenant :
un premier fournisseur de stimulation (50) qui fournit à une peau (S) une première stimulation ;
un second fournisseur de stimulation (60) qui fournit à la peau une seconde stimulation induite par une radio fréquence (RF) et différente en type de la première stimulation ; et
une unité source d'alimentation (47) qui alimente de l'énergie au premier fournisseur de stimulation (50) et au second fournisseur de stimulation (60) à partir d'une source d'alimentation,
l'unité source d'alimentation (47) étant configurée pour être capable d'alimenter individuellement alternativement de l'énergie au premier fournisseur de stimulation (50) et au second fournisseur de stimulation (60) à partir de la source d'alimentation ;
l'appareil de soin de beauté (10) comprenant en outre un premier dispositif chauffant (531) et un second dispositif chauffant (532) qui chauffent la peau (S) à partir d'une surface de la peau (S), où
le premier dispositif chauffant (531) et le second dispositif chauffant (532) sont entraînés à un moment de préchauffage, et
l'un ou l'autre du premier dispositif chauffant (531) et du second dispositif chauffant (532) est entraîné après le préchauffage.

2. Appareil de soin de beauté (10) selon la revendication 1, comprenant en outre :
un dispositif de commande (15) qui commande l'unité source d'alimentation (47) ; et
un circuit de commutation (48) qui surveille la commande du dispositif de commande (15) pour l'unité source d'alimentation (47) et qui effectue la commutation de l'unité source d'alimentation (47) vers un état d'arrêt lorsqu'une anomalie se produit dans la commande.

3. Appareil de soin de beauté (10) selon l'une ou l'autre des revendications 1 et 2,
la première stimulation étant une vibration d'onde ultrasonore, et
une fréquence de vibration de la première stimulation et une fréquence d'un courant de génération de la seconde stimulation coïncidant l'une avec l'autre.
